(19) Europäisches Patentamt
European Patent Office
Office européen des brevets

(11) Veröffentlichungsnummer: **0 134 922**
**B1**

(12) # EUROPÄISCHE PATENTSCHRIFT

(45) Veröffentlichungstag der Patentschrift: **14.12.88**

(51) Int. Cl.⁴: **C 07 D 475/08**, A 61 K 31/505

(21) Anmeldenummer: **84106993.3**

(22) Anmeldetag: **19.06.84**

(54) **Neue 2-Piperazino-pteridine, Verfahren zu ihrer Herstellung und diese Verbindungen enthaltende Arzneimittel.**

(30) Priorität: **02.07.83 DE 3323932**

(43) Veröffentlichungstag der Anmeldung: **27.03.85 Patentblatt 85/13**

(45) Bekanntmachung des Hinweises auf die Patenterteilung: **14.12.88 Patentblatt 88/50**

(84) Benannte Vertragsstaaten: **AT BE CH DE FR IT LI LU NL SE**

(56) Entgegenhaltungen:
**FR-A-1 352 111**
**US-A-2 940 972**

**CHEMICAL ABSTRACTS, Band 85, Nr. 21, 22. November 1976, Columbus, Ohio, USA M.A. KALDRIKYAN et al. "Pteridine derivatives. I. Synthesis of some substituted 6,7-diarylpteridines" Seite 546, Spalte 1, Zusammenfassung-Nr. 160 035y**

(73) Patentinhaber: **Dr. Karl Thomae GmbH, Postfach 1755, D-7950 Biberach (Riss) (DE)**

(72) Erfinder: **Roch, Josef, Dr. Dipl.- Chem., Stecherweg 19, D-7950 Biberach 1 (DE)**
Erfinder: **Nickl, Josef, Dr. Dipl.- Chem., Silcherstrasse 8, D-7950 Biberach 1 (DE)**
Erfinder: **Müller, Erich, Dr. Dipl.- Chem., Talfeldstrasse 34, D-7950 Biberach 1 (DE)**
Erfinder: **Narr, Berthold, Dr. Dipl.- Chem., Obere Au 5, D-7950 Biberach 1 (DE)**
Erfinder: **Weisenberger, Johannes, Dr. Dipl.- Chem., Haydnweg 5, D-7950 Biberach 1 (DE)**
Erfinder: **Zimmermann, Rainer, Dr. Dipl.- Biochem., Laurenbühlstrasse 17, D-7951 Mittelbiberach (DE)**
Erfinder: **Haarmann, Walter, Dr., Schlierholzweg 27, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 134 922 B1

LIBER, STOCKHOLM 1988

**Beschreibung**

In der US-A-2 940 972 werden bereits tetrasubstituierte Pteridine beschrieben, welche wertvolle pharmakologische Eigenschaften aufweisen, nämlich coronarerweiternde, sedative, antipyretische und analgetische Wirkungen.

Es wurde nun gefunden, daß die neuen 2-Piperazino-pteridine der allgemeinen Formel

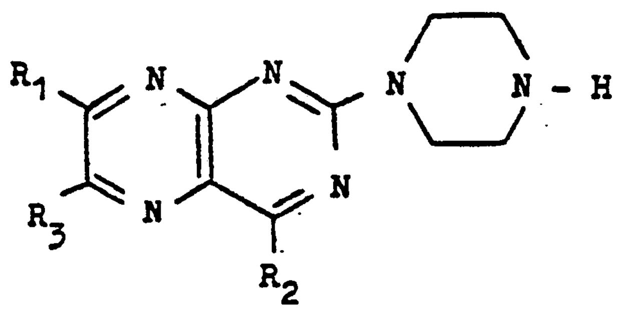

,(I)

und deren Säureadditionssalze, insbesondere deren physiologisch verträgliche Säureadditionssalze mit anorganischen oder organischen Säuren, ebenfalls wertvolle pharmakologische Eigenschaften aufweisen, überraschenderweise jedoch antithrombotische und metastasenhemmende Wirkungen.

In der obigen allgemeinen Formel I bedeutet

$R_1$ eine Phenylalkylamino-, Alkylamino- oder Dialkylaminogruppe, eine Piperidino-, Morpholino-, Thiomorpholino- oder 1-Oxidothiomorpholinogruppe,

$R_2$ eine Dialkylamino-, Piperidino-, Morpholino-, Thiomorpholino- oder 1-Oxidothiomorpholinogruppe und

$R_3$ ein Halogenatom, eine Alkoxy-, Alkylthio-, Phenylalkoxy- oder Phenylalkylthiogruppe, wobei in den obengenannten Resten $R_1$, $R_2$ und $R_3$ jeder Alkylteil jeweils 1 bis 3 Kohlenstoffatome enthalten kann.

Gegenstand der vorliegenden Erfindung sind somit die neuen 2-Piperazino-pteridine der obigen allgemeinen Formel I, deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

Für die bei der Definition der Reste $R_1$ bis $R_3$ eingangs erwähnten Bedeutungen kommt beispielsweise

für $R_1$ die der Methylamino-, Äthylamino-, Propylamino-, Isopropylamino-, Benzylamino-, 1-Phenyläthylamino-, 2-Phenyläthylamino-, 3-Phenylpropylamino-, Dimethylamino-, Diäthylamino-, Dipropylamino-, Methyl-äthylamino-, Piperidino-, Morpholino, Thiomorpholino- oder 1-Oxidothiomorpholinogruppe,

für $R_2$ die der Dimethylamino-, Diäthylamino-, Dipropylamino-, Diisopropylamino-, Methyl-äthylamino-, Äthyl-propylamino-, Piperidino-, Morpholino-, Thiomorpholino- oder 1-Oxidothiomorpholinogruppe und

für $R_3$ die des Chlor- oder Bromatoms, der Methoxy-, Ähthoxy-, Propoxy-, Isopropyoxy, Benzyloxy-, 1-Phenyläthoxy-, 2-Phenyläthoxy-, 1-Phenylpropoxy-, 2-Phenylpropoxy, 3-Phenylpropoxy-, 1-Methyl-2-Phenyläthoxy-, Methylmercapto-, Äthylmercapto-, Propylmercapto-, Isopropylmercapto-, Benzylmercapto-, 1-Phenyläthylmercapto-, 2-Phenyläthylmercapto- oder 3-Phenylpropylmercaptogruppe in Betracht.

Bevorzugte Verbindungen der obigen allgemeinen Formel I sind diejenigen, in denen

$R_1$ eine Dimethylamino-, Benzylamino-, Piperidino- Morpholino-, Thiomorpholino- oder 1-Oxidothiomorpholinogruppe,

$R_2$ eine Dimethylamino-, Piperidino-, Morpholino-, Thiomorpholino- oder 1-Oxidothiomorpholinogruppe und

$R_3$ ein Chlor- oder Bromatom, eine Alkoxy- oder Alkylmercaptogruppe mit jeweils 1 bis 3 Kohlenstoffatomen im Alkylteil, eine Benzyloxy- oder Benzylmercaptogruppe bedeuten, und deren Säureadditionssalze, insbesondere deren physiologisch verträgliche Säureadditionssalze mit anorganischen oder organischen Säuren.

Besonders bevorzugte Verbindungen der obigen allgemeinen Formel I sind jedoch diejenigen, in denen

$R_1$ und $R_2$, die gleich oder verschieden sein können, je eine Dimethylamino-, Morpholino-, Thiomorpholino- oder 1-Oxidothiomorpholinogruppe und $R_1$ auch eine Benzylaminogruppe und

$R_3$ ein Chloratom, eine Alkoxy- oder Alkylmercaptogruppe mit jeweils 1 bis 3 Kohlenstoffatomen im Alkylteil, eine Benzyloxy- oder Benzylmercaptogruppe bedeuten, und deren Säureadditionssalze, insbesondere deren physiologisch verträgliche Säureadditionssalze mit anorganischen oder organischen Säuren.

Erfindungsgemäß erhält man die neuen Verbindungen nach folgenden Verfahren:

a) Zur Herstellung von Verbindungen der allgemeinen Formel I, in der $R_3$ ein Halogenatom darstellt:
Umsetzung einer Verbindung der allgemeinen Formel

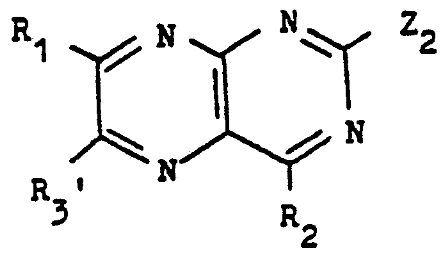

,(II) ,(II)

in der
$R_1$ und $R_2$ wie eingangs definiert sind,
$R_3'$ ein Halogenatom und
$Z_2$ eine nucleophil austauschbare Gruppe wie ein Halogenatom, z. B. ein Chlor- oder Bromatom, bedeuten, mit einem Piperazin der allgemeinen Formel

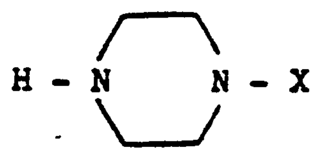

,(III) ,(III)

in der
X ein Wasserstoffatom oder einen hydrolytisch abspaltbaren Schutzrest darstellt, und erforderlichenfalls anschließende Abspaltung des verwendeten Schutzrestes.

Die Umsetzung wird zweckmäßigerweise in einem Lösungsmittel wie Tetrahydrofuran, Dioxan, Benzol, Toluol oder Dimethylglycoläther bei Temperaturen zwischen 50 und 150°C, vorzugsweise bei der Siedetemperatur des verwendeten Lösungsmittels, oder in der Schmelze durchgeführt. Hierbei kann die Verwendung eines säurebindenden Mittels wie Natriumcarbonat, Triäthylamin oder Pyridin von Vorteil sein.

Die gegebenenfalls erforderliche Abspaltung eines verwendeten Schutzrestes erfolgt in Gegenwart einer Säure wie Salzsäure oder Schwefelsäure oder einer Base wie Natriumhydroxid oder Kaliumhydroxid vorzugsweise in einem wässrigen Lösungsmittel wie Methanol/Wasser, Äthanol/Wasser oder Dioxan/Wasser bei Temperaturen bis zur Siedetempertur des verwendeten Lösungsmittels.

b) Zur Herstellung von Verbindungen der allgemeinen Formel I, in der $R_3$ eine Alkoxy-, Alkylmercapto-, Phenylalkoxy- oder Phenylalkylmercaptogruppe darstellt:
Umsetzung einer Verbindung der allgemeinen Formel

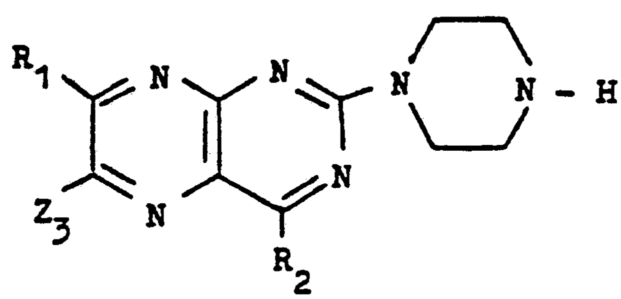

,(IV) ,(IV)

in der
$R_1$ und $R_2$ wie eingangs definiert sind und
$Z_3$ eine nucleophil austauschbare Gruppe wie ein Halogenatom, z. B. ein Chlor- oder Bromatom, darstellt, mit einer Verbindung der allgemeinen Formel

$R_3'$ - H ,(V)

in der
$R_3'$ eine gegebenenfalls durch eine Phenylgruppe substituierte Alkoxy- oder Alkylmercaptogruppe darstellt, wobei der Alkylteil jeweils 1 bis 3 Kohlenstoffatome enthalten kann, oder mit dessen Alkalisalz.

Die Umsetzung wird vorzugsweise in einem geeigneten Lösungsmittel wie Dioxan, Tetrahydrofuran,

Methanol, Äthanol, Propanol, Isopropanol oder Benzylalkohol und vorzugsweise in Gegenwart eines entsprechenden Alkalisalzes einer Verbindung der allgemeinen Formel V, z. B. des Natriummethylats, Natriumäthylats oder Natriumbenzylmercaptids, zweckmäßigerweise bei Temperaturen zwischen 50 und 150° C, z. B. bei der Siedetemperatur des verwendeten Lösungsmittels, durchgeführt.

Die erfindungsgemäß erhaltenen Verbindungen lassen sich in ihre Säureadditionssalze, insbesondere in ihre physiologisch verträglichen Salze mit anorganischen oder organischen Säuren überführen. Als Säuren kommen beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure in Betracht.

Die als Ausgangsstoff verwendeten Verbindungen der allgemeinen Formeln II bis V sind zum größten Teil bekannt bzw. man erhält diese nach dem in der US-A-2 940 972 beschriebenen Verfahren (siehe Beispiele A bis C).

Wie bereits eingangs erwähnt, weisen die neuen Verbindungen der allgemeinen Formel I und deren physiologisch verträgliche Säureadditionssalze mit anorganischen oder organischen Säuren wertvolle pharmakologische Eigenschaften auf, insbesondere jedoch antithrombotische und metastasenhemmende Wirkungen und eine Hemmwirkung auf Phosphodiesterase.

Beispielsweise wurden die Verbindungen

A = 6-Benzylthio-4,7-dimorpholino-2-piperazino-pteridin

B = 6-Chlor-4,7-bis-(dimethylamino)-2-piperazino-pteridin

C = 6-Benzylthio-4,7-bis-(dimethylamino)-2-piperazino-pteridin

D = 7-Benzylamino-6-methylthio-4-(1-oxidothiomorpholino)-2-piperazino-pteridin und

E = 6-Chlor-2-piperazino-4-dimethylamino-7-benzylamino-pteridin

auf ihre Hemmwirkung auf die Phosphodiesterase (PDE) von Tumorzellen und von Humanthrombozyten in vitro in Anlehnung an die von Pöch et al. beschriebenen Methode wie folgt untersucht (siehe Naunyn-Schmiedebergs Arch. Pharmak. 268, 272 - 279 (1971)):

a) Enzymgewinnung:

Die Phosphodiesterase wurde aus B16 Melanomgewebe von Mäusen durch Zentrifugation des Gewebehomogenates bei 5.000 x g (15 min, 4° C) gewonnen. Die Homogenisation der Gewebe erfolgte durch wiederholtes Frieren/Auftauen und Homogenisation nach Potter-Elvehjem bzw. durch Ultraschall. Der die PDE enthaltende Homogenat-Überstand wurde portioniert tiefgefroren und bei -25° C tiefgefroren. Die Gewinnung der PDE aus Humanthrombozyten erfolgte in analoger Weise durch Frieren/Auftauen und Zentrifugation.

b) Bestimmung der PDE-Hemmung (PDE-assay):

Die Bestimmung der PDE-Hemmung durch die Prüfsubstanzen erfolgte mit 1 µmol/1 $^3$H-cAMP als Substrat. Die PDE-Hemmung wurde durch Messung des Abbaus des eingesetzten Substrats $^3$H-cAMP zu $^3$H-AMP im Vergleich zur Kontrolle ohne Prüfsubstanz erfaßt.

Das gebildete $^3$H-AMP wurde durch eine Zinksulfat-Bariumhydroxid-Fällung vom verbliebenen $^3$H-cAMP abgetrennt.

Die Berechnung der $ED_{50}$ als die Konzentration, die die PDE-Aktivität um 50 % hemmte, erfolgte mittels linearer Regressionsanalyse.

| Substanz | PDE-Hemmung ($ED_{50}$) Thrombozyten | B16-Tumorzellen |
|---|---|---|
| A | 0,01 | 0,088 |
| B | 35 | 0,95 |
| C | 10 | 0,88 |
| D | 0,048 | 0,97 |
| E | 14 | 0,37 |

**Akute Toxizität:**

Die orientierende akute Toxizität der zu untersuchenden Substanzen wurde orientierend an Gruppen von je 5 Mäusen nach oraler Gabe einer Einzeldosis bestimmt (Beobachtungszeit: 14 Tage):

| Substanz | Orientierende aktute Toxizität |
|---|---|
| A | > 250 mg/kg (0 von 5 Tieren gestorben) |
| B | > 250 mg/kg (0 von 5 Tieren gestorben) |
| C | > 250 mg/kg (0 von 5 Tieren gestorben) |
| D | > 250 mg/kg (0 von 5 Tieren gestorben) |
| E | > 250 mg/kg (0 von 5 Tieren gestorben) |

Die erfindungsgemäß hergestellten neuen Verbindungen eignen sich aufgrund ihrer oben erwähnten pharmakologischen Eigenschaften zur Prophylaxe thrombo-embolischer Erkrankungen wie Coronarinfarkt, Cerebralinfarkt, sogn. transient ischaemic attacks, Amaurosis fugax, zur Prophylaxe der Arteriosklerose und zur Metastasenprophylaxe.

Die zur Erzielung einer entsprechenden Wirkung erforderlichen Dosierung beträgt zweckmäßigerweise 2- bis 4-mal täglich 0,1 bis 4 mg/kg Körpergewicht, vorzugsweise 0,2 bis 3 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z. B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, nichtionische Tenside wie z. B. Polyoxyäthylen-Fettsäureester, Wasser-Polyäthylenglykol, Propylenglykol, Cetylstearylalkohohl, Carboxymethylcellulose oder fetthaltige Substanzen wie Hartfett oder deren geeignete Gemische, in übliche galenische Zubereitungen wie Tabletten, Dragées, Kapseln, Pulver, Suspensionen, Tropfen, Ampullen, Säfte oder Zäpfchen einarbeiten.

Die nachfolgenden Beispiele sollen die Erfindung näher erläutern:

**Beispiel A**

<u>2,6,7-Trichlor-4-morpholino-pteridin</u>

In eine Suspension aus 13,5 g (0,05 Mol) 2,4,6,7-Tetrachlor-pteridin in etwa 400 ml Chloroform und 10 g (0,1 Mol) Kaliumbicarbonat, gelöst in 100 ml Wasser, wird unter kräftigem Rühren und Kühlen auf -5 bis 0°C eine Lösung von 4,35 g (0,05 Mol) Morpholin in 100 ml Chloroform langsam eingetropft und noch etwa 30 Minuten lang unter Kühlen gerührt. Die das Reakionsprodukt enthaltende Chloroformphase wird abgetrennt, über Natriumsulfat getrocknet und im Vakuum eingedampft.

Ausbeute: 13,5 g (84 % der Theorie),
Schmelzpunkt: 211 - 213°C (Essigsäureäthylester)

Analog Beispiel A werden folgende Verbindungen hergestellt:

2,6,7-Trichlor-4-thiomorpholino-pteridin
Schmelzpunkt: 191 - 193°C

2,6,7-Trichlor-4-(1-oxidothiomorpholino)-pteridin
Schmelzpunkt: 212 - 214°C (Zers)

**Beispiel B**

<u>2,6-Dichlor-4,7-bis-(1-oxidothiomorpholino)-pteridin</u>

In eine Lösung von 13,5 g (0,05 Mol) 2,4,6,7-Tetrachlor-pteridin in 300 ml Dioxan werden unter Rühren bei Raumtemperatur 23,8 g (0,2 Mol) Thiomorpholin-1-oxid, gelöst in 100 ml Dioxan, langsam zugegeben, wobei rasch ein gelblicher Niederschlag ausfällt. Das Reaktionsgemisch wird in etwa 2 l Wasser aufgenommen. Nach einigem Stehen wird das abgeschiedene Reaktionsprodukt abgesaugt und mit Wasser gewaschen und bei etwa 70°C getrocknet.

Ausbeute: 19,2 g (88 % der Theorie),
Schmelzpunkt: 237 - 239°C (Äthanol).

Analog Beispiel B werden folgende Verbindungen hergestellt:

2,6-Dichlor-4,7-dimorpholino-pteridin
Schmelzpunkt: 206 - 208°C

2,6-Dichlor-4,7-bis-(thiomorpholino)-pteridin
Schmelzpunkt: 193 - 195° C (aus Dioxan)

2,6-Dichlor-4,7-bis-(dimethylamino)-pteridin
Schmelzpunkt: 245 - 247° C

2,6-Dichlor-4,7-dipiperidino-pteridin
Schmelzpunkt: 185 - 187° C

**Beispiel C**

7-Benzylamino-2,6-dichlor-4-morpholino-pteridin

Zu einer Suspension von 9,6 g (0,03 Mol) 2,6,7-Trichlor-4-morpholino-pteridin in etwa 150 ml Dioxan gibt man bei Raumtempertur unter Rühren langsam eine Lösung von 7 g (0,065 Mol) Benzylamin in 50 ml Dioxan. Nach etwa einstündigem Rühren wird das Reaktionsgemisch in etwa 1 l Wasser aufgenommen. Der nach einigem Stehen abgeschiedene Niederschlag wird abgesaugt, mit Wasser gewaschen und bei 60° C getrocknet.
Ausbeute: 10,9 g (94 % der Theorie),
Schmelzpunkt: 213 - 214° C (Äthanol/Dioxan 2 : 1)

Analog Beispiel C werden folgende Verbindungen hergestellt:

7-Benzylamino-2,6-dichlor-4-(1-oxidothiomorpholino)-pteridin
Schmelzpunkt: 253 - 254° C

2,6-Dichlor-7-morpholino-4-(1-oxidothiomorpholino)-pteridin
Schmelzpunkt: 215 - 217° C

2,6-Dichlor-4-morpholino-7-(1-oxidothiomorpholino)-pteridin
Schmelzpunkt: 218 - 220° C

**Beispiel 1**

6-Chlor-4,7-dimorpholino-2-piperazino-pteridin

9,3 g (0,025 Mol) 2,6-Dichlor-4,7-dimorpholino-pteridin werden mit 8,6 g (0,1 Mol) wasserfreiem Piperazin in 200 ml Dioxan eine Stunde lang unter Rückfluß erhitzt. Das Lösungsmittel wird weitgehend abdestilliert und der verbleibende Rückstand mit etwa 100 ml Wasser digeriert. Nach kurzem Stehen wird abgesaugt, mit Wasser gewaschen und bei etwa 70° C getrocknet (Schmelzpunkt: 218 - 220° C).
Ausbeute: 8,9 g (85 % der Theorie),
Schmelzpunkt: 220 - 222° C.
$C_{18}H_{25}ClN_8O_2$ (420,9)

| | | | | |
|---|---|---|---|---|
| Ber.: | C 51,36 | H 5,99 | Cl 8,42 | N 26,62 |
| Gef.: | 51,21 | 5,97 | 8,48 | 26,68 |

**Beispiel 2**

6-Benzylthio-4,7-dimorpholino-2-piperazino-pteridin

Zu einer Lösung von 6,3 g (0,015 Mol) 6-Chlor-4,7-dimorpholino-2-piperazino-pteridin in 200 ml Dioxan wird eine Lösung von 0,35 g Natrium und 2 ml (ca. 0,017 Mol) Benzylmercaptan in 100 ml Dioxan Gegeben und anschließend etwa 2 Stunden lang unter Rückfluß erhitzt. Das Lösungsmittel wird im Vakuum weitgehend abdestilliert und der verbleibende Rückstand in etwa 200 ml Wasser aufgenommen. Nach dem Erstarren wird das Reakionsprodukt abgesaugt, mit Wasser gewaschen und bei Raumtempertur im Vakuum getrocknet.
Ausbeute: 6,4 g (84 % der Theorie).
Nach Reinigung über eine Kieselgelsäule (Laufmittel: Methanol/konz. Ammoniak; 50 : 1) und

Umkristallisieren aus Essigsäureäthylester schmilzt die Substanz bei 135 - 137° C.
$C_{25}H_{32}N_8O_2S$ (508,7)

| | | | | |
|---|---|---|---|---|
| Ber.: | C 59,03 | H 6,34 | N 22,03 | S 6,30 |
| Gef.: | 59,28 | 6,55 | 22,19 | 6,36 |

**Beispiel 3**

7-Benzylamino-6-methoxy-4-(1-oxidothiomorpholino)-2-piperazino-pteridin

In eine Lösung von 2,9 g (0,006 Mol) 7-Benzylamino-6-chlor-4-(1-oxidothiomorpholino)-2-piperazino-pteridin in 100 ml Dioxan wird eine Lösung von 0,23 g (0,01 Mol Natrium in 10 ml Methanol eingegossen. Das erhaltene Gemisch wird 30 Minuten lang unter Rückfluß erhitzt und anschließend das Lösungsmittel im Vakuum weitgehend abdestilliert. Der Rückstand wird in etwa 70 ml Wasser aufgenommen und das abgeschiedene Reaktionsprodukt abgesaugt, mit Wasser gewaschen und bei etwa 60° C getrocknet.

Ausbeute: 2,6 g (93 % der Theorie).

Nach Umfällen aus 0,1 n-Salzsäure mittels Ammoniak und Umkristallisieren aus Essigsäureäthylester/Methanol (4 : 1) schmilzt die Verbindung bei 148 - 151° C.
$C_{22}H_{28}N_8O_2S$ (468,6)

| | | | | |
|---|---|---|---|---|
| Ber.: | C 56,39 | H 6,02 | N 23,91 | S 6,84 |
| Gef.: | 56,61 | 6,27 | 23,40 | 6,44 |

**Beispiel 4**

6-Chlor-4-morpholino-7-(1-oxidothiomorpholino)-2-piperazino-pteridin

Hergestellt analog Beispiel 1 aus 2,6-Dichlor-4-morpholino-7-(1-oxidothiomorpholino)-pteridin und Piperazin
Schmelzpunkt: 225 - 227° C (Umfällung aus 0,1 n-HCl mittels Ammoniak).

**Beispiel 5**

6-Chlor-4,7-bis-(1-oxidothiomorpholino)-2-piperazino-pteridin

Hergestellt analog Beispiel 1 aus 2,6-Dichlor-4,7-bis-(1-oxidothiomorpholino)-pteridin und Piperazin.
Schmelzpunkt: > 200° C (Zersetzung).

**Beispiel 6**

6-Chlor-4,7-dipiperidino-2-piperazino-pteridin

Hergestellt analog Beispiel 1 aus 2,6-Dichlor-4,7-dipiperidino-pteridin und Piperazin.
Schmelzpunkt: bei etwa 200° C Zersetzung.

**Beispiel 7**

6-Chlor-4,7-bis-(dimethylamino)-2-piperazino-pteridin

Hergestellt analog Beispiel 1 aus 2,6-Dichlor-4,7-bis-(dimethylamino)-pteridin und Piperazin.
Schmelzpunkt: 130 - 134° C.

**Beispiel 8**

6-Chlor-2-piperazino-4,7-bis-(thiomorpholino)-pteridin

Hergestellt analog Beispiel 1 aus 2,6-Dichlor-4,7-bis-(thiomorpholino)-pteridin und Piperazin. Schmelzpunkt: 194 - 196° C (Essigsäureäthylester )

**Beispiel 9**

6-Chlor-7-morpholino-4-(1-oxidothiomorpholino)-2-piperazino-pteridin

Hergestellt analog Beispiel 1 aus 2,6-Dichlor-7-morpholino-4-(1-oxidothiomorpholino)-pteridin und Piperazin. Schmelzpunkt: > 240° C Zersetzung.

**Beispiel 10**

7-Benzylamino-6-chlor-4-morpholino-2-piperzino-pteridin

Hergestellt analog Beispiel 1 aus 7-Benzylamino-2,6-dichlor-4-morpholino-pteridin und Piperazin. Schmelzpunkt: 195 - 197° C (Methanol/Wasser).

**Beispiel 11**

7-Benzylamino-6-chlor-4-(1-oxidothiomorpholino)-2-piperazino-pteridin

Hergestellt analog Beispiel 1 aus 7-Benzylamino-2,6-dichlor-4-(1-oxidothiomorpholino)-pteridin und Piperazin. Schmelzpunkt: > 200° C Zersetzung.

**Beispiel 12**

6-Benzylthio-4,7-bis-(dimethylamino)-2-piperazino-pteridin

Hergestellt analog Beispiel 2 aus 6-Chlor-4,7-bis-(dimethylamino)-2-piperazino-pteridin und Benzylmercaptan. Schmelzpunkt: 150 - 152° C.

**Beispiel 13**

7-Benzylamino-6-methylthio-4-(1-oxidothiomorpholino)-2-piperazino-pteridin

Hergestellt analog Beispiel 2 aus 7-Benzylamino-6-chlor-4-(1-oxidothiomorpholino)-2-piperazino-pteridin und Methylmercaptan. Schmelzpunkt des Hydrochlorids: 159 - 162° C.

**Beispiel 14**

4-Morpholino-7-(1-oxidothiomorpholino)-2-piperazino-6-propylthio-pteridin

Hergestellt analog Beispiel 2 aus 6-Chlor-4-morpholino-7-(1-oxidothiomorpholino)-2-piperazino-pteridin und Propylmercaptan. Schmelzpunkt: 125 - 130° C.

**Beispiel 15**

7-Benzylamino-6-benzylthio-4-(1-oxidothiomorpholino)-2-piperazino-pteridin

Hergestellt analog Beispiel 2 aus 7-Benzylamino-6-chlor-4-(1-oxidothiomorpholino)-2-piperazino-pteridin und Benzylmercaptan.

Schmelzpunkt: > 160° C (Zersetzung).

**Beispiel 16**

6-Äthoxy-2-piperazino-4,7-bis-(thiomorpholino)-pteridin

Hergestellt analog Beispiel 3 aus 6-Chlor-2-piperazino-4,7-bis-(thiomorpholino)-pteridin und Äthanol)

Schmelzpunkt: 147 - 151° C.

**Beispiel 17**

6-Benzyloxy-4,7-bis-(dimethylamino)-2-piperazino-pteridin

Hergestellt analog Beispiel 3 aus 6-Chlor-4,7-bis-(dimethylamino)-2-piperazino-pteridin und Benzylalkohol.

Schmelzpunkt: 166 - 168° C.

**Beispiel 18**

6-Chlor-2-piperazino-4-dimethylamino-7-benzylamino-pteridin

Hergestellt analog Beispiel 1 aus 2,6-Dichlor-4-dimethylamino-7-benzylamino-pteridin und Piperazin.

Schmelzpunkt: 134 - 137° C.

**Beispiel 19**

6-Chlor-2-piperazino-4-thiomorpholino-7-benzylamino-pteridin

Hergestellt analog Beispiel 1 aus 2,6-Dichlor-4-thiomorpholino-7-benzylamino-pteridin und Piperazin.

Schmelzpunkt: 160 - 165° C.

**Beispiel 20**

6-Chlor-2-piperazino-4-thiomorpholino-7-dimethylamino-pteridin

Hergestellt analog Beispiel 1 aus 2,6-Dichlor-4-thiomorpholino-7-dimethylamino-pteridin und Piperazin.

Schmelzpunkt: 205 - 207° C.

**Beispiel 21**

7-Benzylamino-6-benzylthio-2-piperazino-4-thiomorpholino-pteridin

Hergestellt analog Beispiel 2 aus 7-Benzylamino-6-chlor-2-piperazino-4-thiomorpholino-pteridin.

Schmelzpunkt: ab 70° C (sintern)

**Beispiel A**

Dragées mit 4 mg 6-Benzylthio-4,7-dimorpholino-2-piperazino-pteridin

Zusammensetzung:
1 Dragéekern enthält:

| | | |
|---|---|---|
| Wirksubstanz | (1) | 4,0 mg |
| Milchzucker | (2) | 27,0 mg |
| Maisstärke | (3) | 14,5 mg |
| Polyvinylpyrrolidon | (4) | 4,0 mg |
| Magnesiumstearat | (5) | 0,5 mg |
| | | 50,0 mg |

**Herstellung:**

Die Stoffe 1-3 werden mit einer wäßrigen Lösung von 4 gleichmäßig befeuchtet, durch 1 mm-Maschenweite gesiebt, getrocknet und erneut durch 1 mm-Maschenweite gesiebt. Nach Zumischen von 5 wird die Mischung zu Dragéekernen verpreßt.

Dragéekerne 5 mm 0, bikonvex, rund

**Dragierung:**

Übliche Zuckerdragierung auf 70 mg Endgewicht:

**Beispiel B**

Tabletten mit 8 mg 6-Benzylthio-4,7-dimorpholino-piperazino-pteridin

1 Tablette enthält:

| | |
|---|---|
| Wirksubstanz | 8,0 mg |
| Milchzucker | 23,0 mg |
| Maisstärke | 14,5 mg |
| Polyvinylpyrrolidon | 4,0 mg |
| Magnesiumstearat | 0,5 mg |
| | 50,0 mg |

**Herstellung:**

Analog den Dragéekernen.

**Tablettenbeschreibung:**

Gewicht: 50 mg
Durchmesser: 5 mm, biplan, beidseitige Facette

**Beispiel C**

Suppositorien zu 25 mg 6-Benzylthio-4,7-dimorpholino-2-piperazino-pteridin

1 Zäpfchen enthält:

| | |
|---|---|
| Wirksubstanz | 0,025 g |
| Hartfett (z. B. Witepsol H 19 und Witepsol H 45) | 1,675 g |
| | 1,700 g |

**Herstellung:**

Das Hartfett wird geschmolzen. Bei 38°C wird die gemahlene Wirksubstanz in der Schmelze homogen dispergiert. Es wird auf 35°C abgekühlt und in schwach vorgekühlte Suppositorien formen ausgegossen.

Zäpfchengewicht: 1,7 g

**Beispiel D**

Suspension mit 8 mg 6-Benzylthio-4,7-dimorpholino-2-piperazino-pteridin

100 ml Suspension enthalten:

| | | |
|---|---|---|
| Wirksubstanz | | 0,16 g |
| Carboxymethylcellulose | | 0,1 g |
| p-Hydroxybenzoesäuremethylester | | 0,05 g |
| p-Hydroxybenzoesäurepropylester | | 0,01 g |
| Rohrzucker | | 10,0 g |
| Glycerin | | 5,0 g |
| Sorbitlösung 70 % | | 20,0 g |
| Aroma | | 0,3 g |
| Wasser dest. | ad | 100.0 ml |

**Herstellungsverfahren:**

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

**Beispiel E**

Tabletten mit 100 mg 6-Benzylthio-4,7-dimorpholino-2-piperazino-pteridin

Zusammensetzung:

1 Tablette enthält:

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

**Herstellungsverfahren:**

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel

zugemischt. Die preßfertige Mischung wird zu Tabletten verarbeitet.

Tablettengewicht: 220 mg
Durchmesser: 10 mm, biplan mit beidseitiger Facette und einseitiger Teilkerbe.

**Beispiel F**

Hartgelatine-Kapseln mit 150 mg 6-Benzylthio-4,7-dimorpholino-2-piperazino-pteridin

1 Kapsel enthält:

| | | |
|---|---|---|
| Wirkstoff | | 150,0 mg |
| Maisstärke getr. | ca. | 180,0 mg |
| Milchzucker pulv. | ca. | 87,0 mg |
| Magniesiumstearat | | 3,0 mg |
| | ca. | 420,0 mg |

**Herstellung:**

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.

Kapselfüllung: ca. 420 mg
Kapselhülle: Hartgelatine-Kapsel Größe 1.

**Beispiel G**

Suppositorien mit 150 mg 6-Benzylthio-4,7-dimorpholino-2-piperazino-pteridin

1 Zäpfchen enthält:

| | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

**Herstellung:**

Nach dem Aufschmelzen der Suppositorienmasse wird der Wirkstoff darin homogen verteilt und die Schmelze in vorgekühlte Formen gegossen.

**Beispiel H**

Suspension mit 50 mg 6-Benzylthio-4,7-dimorpholino-2-piperazino-pteridin pro 5 ml

100 ml Suspension enthalten:

| | | |
|---|---|---|
| Wirkstoff | | 1,0 g |
| Carboxymethylcellulose-Na-Salz | | 0,1 g |
| p-Hydroxybenzoesäuremethylester | | 0,05 g |
| p-Hydroxybenzoesäurepropylester | | 0,01 g |
| Rohrzucker | | 10,0 g |
| Glycerin | | 5,0 g |
| Sorbitlösung 70 %-ig | | 20,0 g |
| Aroma | | 0,3 g |
| Wasser dest. | ad | 100 ml |

**Herstellung:**

Dest. Wasser wird auf 70°C erhitzt. Hierin wird unter Rühren p-Hydroxybenzoesäuremethylester und -propylester wobei Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

**Beispiel I**

Tabletten mit 150 mg 6-Benzylthio-4,7-dimorpholino-2-piperazino-pteridin

Zusammensetzung:
1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

**Herstellung:**

Die mit Milchzucker, Maisstärke und Kieselsäure gemischte Wirksubstanz wird mit einer 20 %-igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

Tablettengewicht: 300 mg
Stempel: 10 mm, flach

**Beispiel K**

Dragées mit 75 mg 6-Benzylthio-4,7-dimorpholino-2-piperazino-pteridin

1 Drageékern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

**Herstellung:**

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

Kerngewicht: 230 mg
Stempel: 9 mm, gewölbt

Die so hergestellten Dragéekerne werden mit einem Film überzogen, der im wesentlichen aus Hydroxypropylmethylcellulose besteht. Die fertigen Filmdragées werden mit Bienenwachs geglänzt.

Dragéegewicht: 245 mg.

Selbstverständlich können alle übrigen Verbindungen der allgemeinen Formel I als Wirkstoffe in den vorstehenden galenischen Zubereitungen eingesetzt werden.

## Patentansprüche

1. 2-Piperazino-pteridine der allgemeinen Formel

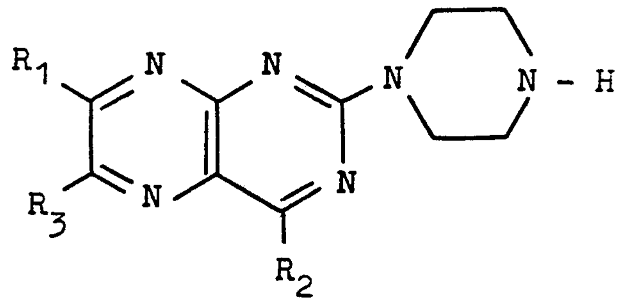

,(I)

in der
$R_1$ eine Phenylalkylamino-, Alkylamino- oder Dialkylaminogruppe, eine Piperidino-, Morpholino-, Thiomorpholino- oder 1-Oxidothiomorpholinogruppe,
$R_2$ eine Dialkylamino-, Piperidino-, Morpholino-, Thiomorpholino- oder 1-Oxidothiomorpholinogruppe und
$R_3$ ein Halogenatom, eine Alkoxy-, Alkylthio-, Phenylalkoxy- oder Phenylalkylthiogruppe, wobei in den obengenannten Resten $R_1$, $R_2$ und $R_3$ jeder Alkylteil jeweils 1 bis 3 Kohlenstoffatome enthalten kann, bedeuten und deren Säureadditionssalze.

2. 2-Piperazino-pteridine der allgemeinen Formel I gemäß Anspruch 1, in der
$R_1$ eine Dimethylamino-, Benzylamino-, Piperidino-, Morpholino-, Thiomorpholino- oder 1-Oxidothiomorpholinogruppe,
$R_2$ eine Dimethylamino-, Piperidino-, Morpholino-, Thiomorpholino- oder 1-Oxidothiomorpholinogruppe und
$R_3$ ein Chlor- oder Bromatom, eine Alkoxy- oder Alkylmercaptogruppe mit jeweils 1 bis 3 Kohlenstoffatomen im Alkylteil, eine Benzyloxy- oder Benzylmercaptogruppe bedeuten, und deren Säureadditionssalze.

3. 2-Piperazino-pteridine der allgemeinen Formel I gemäß Anspruch 1, in der
$R_1$ und $R_2$, die gleich oder verschieden sein können, je eine Dimethylamino-, Morpholino-, Thiomorpholino- oder 1-Oxidothiomorpholinogruppe und $R_1$ auch eine Benzylaminogruppe und
$R_3$ ein Chloratom, eine Alkoxy- oder Alkylmercaptogruppe mit jeweils 1 bis 3 Kohlenstoffatomen im Alkylteil, eine Benzyloxy- oder Benzylmercaptogruppe bedeuten, und deren Säureadditionssalze.

4. 2-Piperazino-pteridine der allgemeinen Formel I gemäß Anspruch 1, in der
$R_1$ und $R_2$ je eine Dimethylamino-, Morpholino- oder 1-Oxidothiomorpholinogruppe und $R_1$ auch eine Benzylaminogruppe und
$R_3$ ein Chloratom, eine Methylmercapto- oder Benzylmercaptogruppe bedeuten, und deren Säureadditionssalze.

5. 6-Benzylthio-4,7-dimorpholino-2-piperazino-pteridin und dessen Säureadditionssalze.

6. 6-Chlor-4,7-bis-(dimethylamino)-2-piperazino-pteridin und dessen Säureadditionssalze.

7. 6-Benzylthio-4,7-bis-(dimethylamino)-2-piprazino-pteridin und dessen Säureadditionssalze.

8. Physiologisch verträgliche Säureadditionssalze der Verbindungen gemäß den Ansprüchen 1 bis 7 mit anorganischen oder organischen Säuren.

9. Arzneimittel, enthaltend eine Verbindung gemäß den Ansprüchen 1 bis 7 oder ein physiologisch verträgliches Säureadditionssalz hiervon neben einem oder mehreren inerten Trägerstoffen- und/oder Verdünnungsmitteln.

10. Verfahren zur Herstellung von 2-Piperazino-pteridinen der allgemeinen Formel

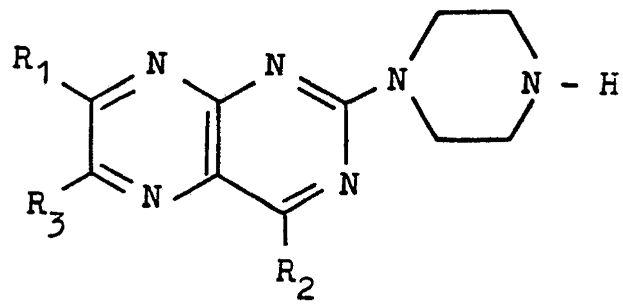

,(I)

in der

$R_1$ eine Phenylalkylamino-, Alkylamino- oder Dialkylaminogruppe, eine Piperidino-, Morpholino-, Thiomorpholino- oder 1-Oxidothiomorpholinogruppe,

$R_2$ eine Dialkylamino-, Piperidino-, Morpholino-, Thiomorpholino- oder 1-Oxidothiomorpholinogruppe und

$R_3$ ein Halogenatom, eine Alkoxy-, Alkylthio-, Phenylalkoxy- oder Phenylalkylthiogruppe, wobei in den obengenannten Resten $R_1$, $R_2$ und $R_3$ jeder Alkylteil jeweils 1 bis 3 Kohlenstoffatome enthalten kann,

bedeuten und von deren Säureadditionssalzen, insbesondere von deren physiologisch verträglichen Säureadditionssalzen mit anorganischen oder organischen Säuren, dadurch gekennzeichnet, daß

a) zur Herstellung von Verbindungen der allgemeinen Formel I, in der $R_3$ ein Halogenatom darstellt, eine Verbindung der allgemeinen Formel

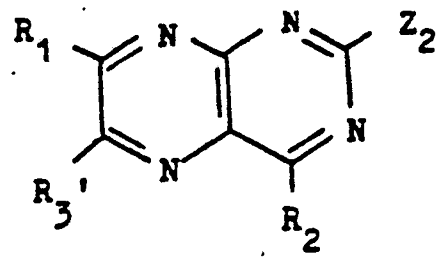

,(II)

,(II)

in der

$R_1$ und $R_2$ wie eingangs definiert sind,

$R_3$ ein Halogenatom und

$Z_2$ eine nucleophil austauschbare Gruppe wie ein Halogenatom bedeuten, mit einem Piperazin der allgemeinen Formel

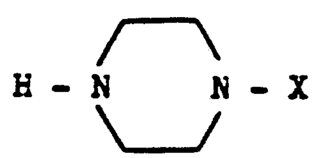

,(III)

,(III)

in der

X ein Wasserstoffatom oder einen hydrolytisch abspaltbaren Schutzrest darstellt, umgesetzt und erforderlichenfalls anschließend der verwendete Schutzrest abgespalten wird oder

b) zur Herstellung von Verbindungen der allgemeinen Formel I, in der $R_3$ eine Alkoxy-, Alkylmercapto-, Phenylalkoxy- oder Phenylalkylmercaptogruppe darstellt, eine Verbindung der allgemeinen Formel

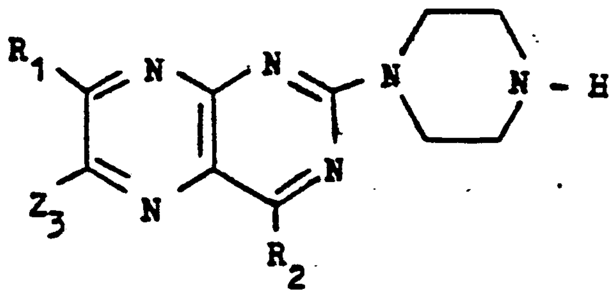

,(IV)

, (IV)

in der

$R_1$ und $R_2$ wie eingangs definiert sind und

$Z_3$ eine nucleophil austauschbare Gruppe wie ein Halogenatom darstellt, mit einer Verbindung der allgemeinen Formel

$R_3'$ - H ,(V)

in der

$R_3'$ eine gegebenenfalls durch eine Phenylgruppe substituierte Alkoxy- oder Alkylmercaptogruppe darstellt, wobei der Alkylteil jeweils 1 bis 3 Kohlenstoffatome enthalten kann, oder mit dessen Alkalisalz umgesetzt wird

und gewünschtenfalls anschließend eine so erhaltene Verbindung der allgemeinen Formel I in ihr Säureadditionssalz, insbesondere in ihr physiologisch verträgliches Säureadditionssalz mit einer anorganischen oder organischen Säure, übergeführt wird.

11. Verwendung einer Verbindung nach mindestens einem der Ansprüche 1 bis 8 zur Herstellung eines Arzneimittels, welches zur Prophylaxe thrombo-embolischer Erkrankungen, zur Prophylaxe der Arteriosklerose und zur Metastasenprophylaxe geeignet ist.

12. Verfahren zur Herstellung eines Arzneimittels gemäß Anspruch 9, dadurch gekennzeichnet, daß eine Verbindung nach mindestens einem der Ansprüche 1 bis 8 in einen oder mehrere inerte Trägerstoffe und/oder Verdünnungsmittel eingearbeitet wird.

**Claims**

1. 2-Piperazino-pteridines of general formula

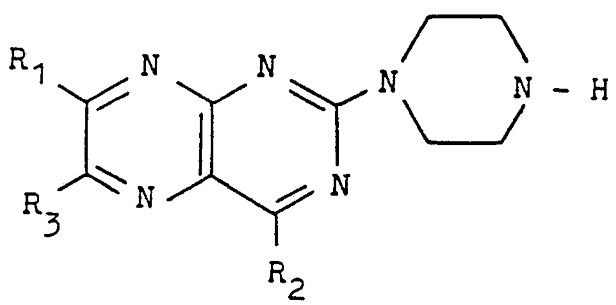

(I)

wherein

$R_1$ represents a phenylalkylamino, alkylamino or dialkylamino group, a piperidino, morpholino, thiomorpholino or 1-oxidothiomorpholino group,

$R_2$ represents a dialkylamino, piperidino, morpholino, thiomorpholino or 1-oxidothiomorpholino group and

$R_3$ represents a halogen atom, an alkoxy, alkylthio, phenylalkoxy or phenylalkylthio group, whilst in the above groups $R_1$, $R_2$ and $R_3$ the alkyl moiety may contain from 1 to 3 carbon atoms, and the acid addition salts thereof.

2. 2-Piperazino-pteridines of general formula I as claimed in claim 1, wherein

$R_1$ represents a dimethylamino, benzylamino, piperidino, morpholino, thiomorpholino or 1-oxidothiomorpholino group,

$R_2$ represents a dimethylamino, piperidino, morpholino, thiomorpholino or 1-oxidothiomorpholino group and

$R_3$ represents a chlorine or bromine atom, an alkoxy or alkylmercapto group with 1 to 3 carbon atoms in the alkyl moiety, or a benzyloxy or benzylmercapto group, and the acid addition salts thereof.

3. 2-Piperazino-pteridines of general formula I as claimed in claim 1, wherein

$R_1$ and $R_2$, which may be the same or different, each represent a dimethylamino, morpholino, thiomorpholino or 1-oxidothiomorpholino group and $R_1$ may also represent a benzylamino group and

$R_3$ represents a chlorine atom, an alkoxy or alkylmercapto group with 1 to 3 carbon atoms in the alkyl moiety or a benzyloxy or benzylmercapto group, and the acid addition salts thereof.

4. 2-Piperazino-pteridines of general formula I as claimed in claim 1, wherein

$R_1$ and $R_2$ each represent a dimethylamino, morpholino or 1-oxidothiomorpholino group and $R_1$ also represents a benzylamino group and

$R_3$ represents a chlorine atom or a methylmercapto or benzylmercapto group, and the acid addition salts thereof.

5. 6-Benzylthio-4,7-dimorpholino-2-piperazino-pteridine and the acid addition salts thereof.

6. 6-Chloro-4,7-bis-(dimethylamino)-2-piperazino-pteridine and the acid addition salts thereof.

7. 6-Benzylthio-4,7-bis-(dimethylamino)-2-piperazino-pteridine and the acid addition salts thereof.

8. Physiologically acceptable acid addition salts of the compounds as claimed in claims 1 to 7 with inorganic or organic acids.

9. Pharmaceutical compositions containing a compound as claimed in claims 1 to 7 or a physiologically acceptable acid addition salt thereof together with one or more inert carriers and/or diluents.

10. Process for the preparation of 2-piperazino-pteridines of general formula

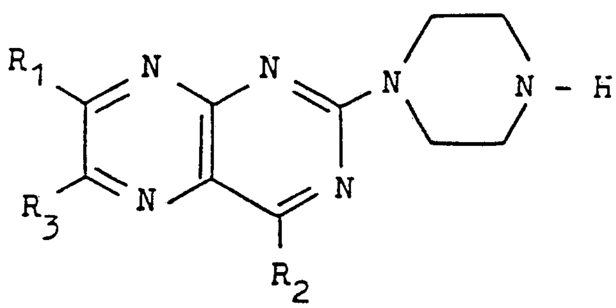

(I)

wherein

$R_1$ represents a phenylalkylamino, alkylamino or dialkylamino group, a piperidino, morpholino, thiomorpholino or 1-oxidothiomorpholino group,

$R_2$ represents a dialkylamino, piperidino, morpholino, thiomorpholino or 1-oxidothiomorpholino group and

$R_3$ represents a halogen atom, an alkoxy, alkylthio, phenylalkoxy or phenylalkylthio group, whilst in the above groups $R_1$, $R_2$ and $R_3$ the alkyl moiety may contain from 1 to 3 carbon atoms, and the acid addition salts thereof, particularly the physiologically acceptable acid addition salts thereof with inorganic or organic acids, characterised in that

a) in order to prepare compounds of general formula I wherein $R_3$ represents a halogen atom, a compound of general formula

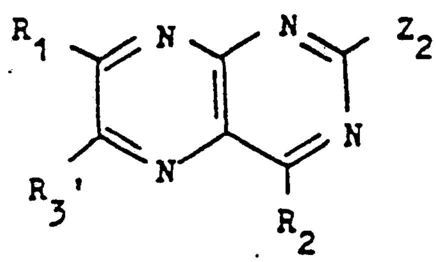

(II)

wherein

$R_1$ and $R_2$ are as hereinbefore defined,

$R_3'$ represents a halogen atom and

$Z_2$ represents a nucleophilically exchangeable group such as a halogen atom,

is reacted with a piperazine of general formula

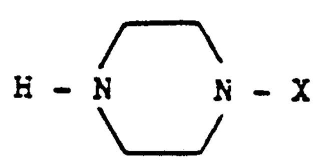

(III)

wherein

X represents a hydrogen atom or a hydrolytically removable protecting group, and subsequently, if necessary, the protecting group used is split off, or

b) in order to prepare compounds of general formula I wherein $R_3$ represents an alkoxy, alkylmercapto, phenylalkoxy or phenylalkylmercapto group, a compound of general formula

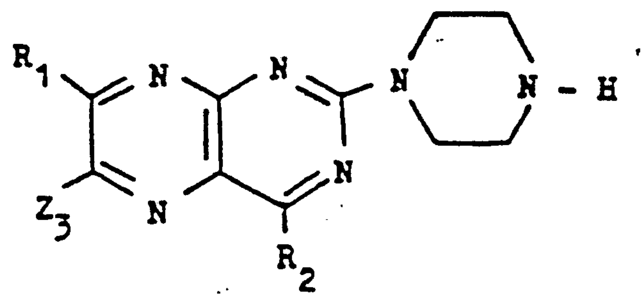

(IV)

wherein

$R_1$ and $R_2$ are as hereinbefore defined and

$Z_3$ represents a nucleophilically exchangeable group such as a halogen atom,

is reacted with a compound of general formula

$R_3' - H$ (V)

wherein

$R_3'$ represents an alkoxy or alkylmercapto group optionally substituted by a phenyl group, wherein the alkyl moiety may contain 1 to 3 carbon atoms, or with an alkali metal salt thereof,

and subsequently, if desired, a compound of general formula I thus obtained is converted into an acid addition salt thereof, particularly a physiologically acceptable acid addition salt with an inorganic or organic acid.

11. Use of a compound as claimed in at least one of claims 1 to 8 for the preparation of a pharmaceutical composition which is suitable for the prophylaxis of thromboembolic disorders, arteriosclerosis and metastasis.

12. Process for preparing a pharmaceutical composition as claimed in claim 9, characterised in that a compound as claimed in at least one of claims 1 to 8 is incorporated in one or more inert carriers and/or diluents.

**Revendications**

1. 2-pipérazino-ptéridines répondant à la formule générale

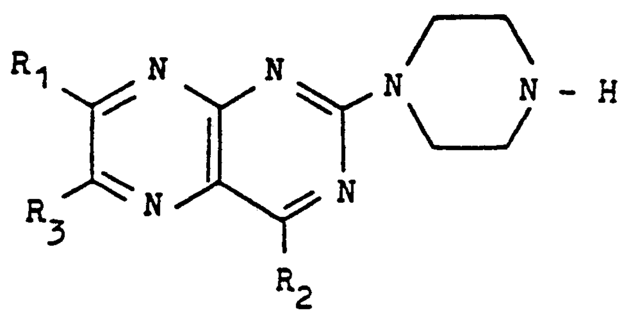

(I)

dans laquelle

$R_1$ désigne un groupe phénylalkylamino, alkylamino ou dialkylamino, un groupe pipéridino, morpholino, thiomorpholino ou l'oxydothiomorpholino,

$R_2$ désigne un groupe dialkylamino, pipéridino, morpholino, thiomorpholino ou l'oxydothiomorpholino, et

$R_3$ désigne un atome d'halogène, un groupe alcoxy, alkylthio, phénylalcoxy ou phénylalkylthio,

chaque partie alkyle, dans les radicaux $R_1$, $R_2$ et $R_3$ précités, pouvant contenir 1 à 3 atomes de carbone, et leurs sels d'addition aux acides.

2. 2-pipérazino-ptéridines répondant à la formule générale I, suivant la revendication 1, dans laquelle

$R_1$ désigne un groupe diméthylamino, benzylamino, pipéridino, morpholino, thiomorpholino ou 1-oxydothiomorpholino,

$R_2$ désigne un groupe diméthylamino, pipéridino, morpholino, thiomorpholino ou l'oxydothiomorpholino, et

$R_3$ désigne un atome de chlore ou de brome, un groupe alcoxy ou alkylmercapto ayant chacun 1 à 3 atomes

de carbone dans la partie alkyle, un groupe benzyloxy ou benzylmercaptan, et leurs sels d'addition aux acides.

3. 2-pipérazino-ptéridines répondant à la formule générale I suivant la revendication 1, dans laquelle

$R_1$ et $R_2$, qui peuvent être identiques ou différents, désignent chacun un groupe diméthylamino, morpholino, thiomorpholino ou 1-oxydothiomorpholino et $R_1$ également un groupe benzylamino, et

$R_3$ désigne un atome de chlore, un groupe alcoxy ou alkylmercapto ayant à chaque fois 1 à 3 atomes de carbone dans la partie alkyle, un groupe benzyloxy ou benzylmercapto, et leurs sels d'addition aux acides.

4. 2-pipérazino-ptéridines répondant à la formule générale I suivant la revendication 1, dans laquelle $R_1$ et $R_2$ désignent chacun un groupe diméthylamino, morpholino ou 1-oxydothiomorpholino et $R_1$ désigne aussi un groupe benzylamino, et

$R_3$ désigne un atome de chlore, un groupe mercapto ou benzylmercapto, et leurs sels d'addition aux acides.

5. 6-benzylthio-4,7-dimorpholino-2-pipérazino-ptéridine et ses sels d'addition aux acides.

6. 6-chloro-4,7-bis-(diméthylamino)-2-pipérazino-ptéridine et ses sels d'addition aux acides.

7. 6-benzylthio-4,7-bis-(diméthylamino)-2-pipérazino-ptéridine et ses sels d'addition aux acides.

8. Sels d'addition aux acides physiologiquement acceptables des composés suivants les revendications 1 à 7 avec des acides minéraux ou organiques.

9. Médicaments contenant un composé suivant les revendications 1 à 7 ou un de ses sels physiologiquement acceptables avec un ou plusieurs supports ou diluants inertes.

10. Procédé de préparation de 2-pipérazino-ptéridines répondant à la formule générale

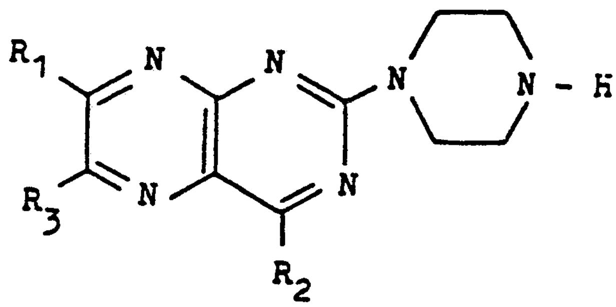

(I)

dans laquelle

$R_1$ désigne un groupe phénylalkylamino, alkylamino ou dialkylamino, un groupe pipéridino, morpholino, thiomorpholino ou 1-oxydothiomorpholino,

$R_2$ désigne un groupe dialkylamino, pipéridino, morpholino, thiomorpholino ou 1-oxydothiomorpholino, et

$R_3$ désigne un atome d'halogène, un groupe alcoxy, alkylthio, phénylalcoxy ou phénylalkylthio, chacune des parties alkyle dans les radicaux $R_1$, $R_2$ et $R_3$ précités pouvant contenir à chaque fois 1 à 3 atomes de carbone,

et de leurs sels d'addition aux acides, en particulier de leurs sels d'addition aux acides physiologiquement acceptables avec des acides minéraux ou organiques, caractérisé en ce que

a) pour la préparation de composés répondant à la formule générale I, dans laquelle $R_3$ désigne un atome d'halogène, on fait réagir un composé répondant à la formule générale

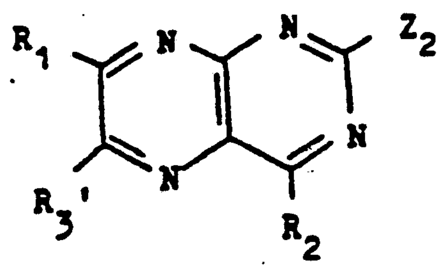

(II)

dans laquelle

$R_1$ et $R_2$ sont tels que définis initialement,

$R_3'$ désigne un atome d'halogène, et

$Z_2$ désigne un groupe échangeable par un mécanisme nucléophile tel qu'un atome d'halogène, avec une pipérazine répondant à la formule générale

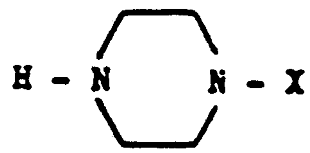

(III)

dans laquelle

X désigne un atome d'hydrogène ou un radical protecteur éliminable par hydrolyse, et on élimine ensuite si nécessaire le radical protecteur utilisé, ou

b) pour la préparation de composés répondant à la formule générale I, dans laquelle $R_3$ représente un groupe alcoxy, alkylmercapto, phénylalcoxy ou phénylalkylmercapto, on fait réagir un composé répondant à la formule générale

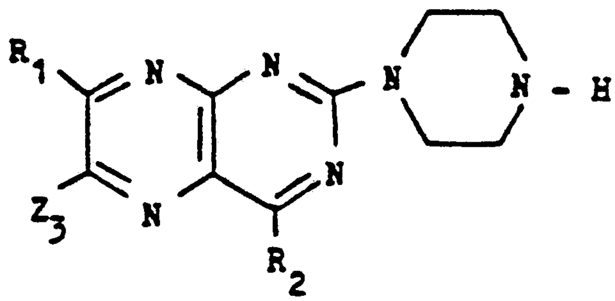

(IV)

dans laquelle

$R_1$ et $R_2$ sont tels que définis initialement et

$Z_3$ représente un groupe échangeable par un mécanisme nucléophile tel qu'un atome d'halogène, avec un composé répondant à la formule générale

$R_3' - H$ (V)

dans laquelle

$R_3'$ représente un groupe alcoxy ou alkylmercapto substitué le cas échéant par un groupe phényle, la partie alkyle pouvant contenir à chaque fois 1 à 3 atomes de carbone, ou avec son sel alcalin,

et si on le désire, on transforme ensuite un composé répondant à la formule générale I ainsi obtenu en son sel d'addition aux acides, en particulier en son sel d'addition aux acides physiologiquement acceptable avec un acide minéral ou organique.

11. Utilisation d'un composé suivant au moins une des revendications 1 à 8 pour la préparation d'un médicament qui est approprié à la prophylaxie des maladies thrombo-emboliques, à la prophylaxie de l'artériosclérose et à la prophylaxie des métastases.

12. Procédé de préparation d'un médicament suivant la revendication 9, caractérisé en ce qu'on incorpore un composé suivant au moins l'une des revendications 1 à 8 à un ou plusieurs supports et/ou diluants inertes.